# EUROPEAN PATENT SPECIFICATION

(11) **EP 2 682 578 B1**
(45) Date of publication and mention of the grant of the patent: **02.03.2016**
(21) Application number: 11859899.4
(22) Date of filing: 03.03.2011
(51) Int. Cl.: F01N 3/08, F01N 3/20

(54) **CATALYST DETERIORATION DETERMINATION SYSTEM**
SYSTEM ZUR BESTIMMUNG VON KATALYSATORVERSCHLEISS
SYSTÈME DE DÉTERMINATION DE DÉTÉRIORATION D'UN CATALYSEUR

(43) Date of publication of application: 08.01.2014
(73) Proprietor: TOYOTA JIDOSHA KABUSHIKI KAISHA, Toyota-shi, Aichi-ken 471-8571 (JP)
(72) Inventor: NAKAMURA, Yoshitaka, Toyota-shi, Aichi-ken 471-8571 (JP); SAITO, Hirotaka, Toyota-shi, Aichi-ken 471-8571 (JP); KIDOKORO, Toru, Toyota-shi, Aichi-ken 471-8571 (JP); SAWADA, Hiroshi, Toyota-shi, Aichi-ken 471-8571 (JP); SAKURAI, Kenji, Toyota-shi, Aichi-ken 471-8571 (JP)
(74) Representative: D Young & Co LLP
(86) International application number: PCT/JP2011/054919
(87) International publication number: WO 2012/117553

(56) References cited:
- EP-A1- 1 225 323
- EP-A1- 2 119 882
- EP-A2- 1 026 374
- EP-A2- 1 519 015
- WO-A1-2008/029236
- JP-A- 2008 057 404
- JP-A- 2008 215 315
- JP-A- 2010 174 814
- JP-A- 2010 203 320

## Description

### [TECHNICAL FIELD]

The present invention relates to a catalyst deterioration determination system.

### [BACKGROUND ART]

NOx in an exhaust gas discharged when an internal combustion engine is in a lean burn condition can be stored by a NOx storage reduction catalyst (hereinafter simply also referred to as a NOx catalyst), and then, the NOx thus stored can be caused to release from the NOx catalyst and to be reduced to N₂ by making an air fuel ratio rich temporarily. In addition, there has been known a technique in which a peak value of an output of an air fuel ratio sensor is obtained at the time of rich burn combustion, a NOx storage capacity of the NOx catalyst is estimated based on the peak value, and the deterioration of the NOx catalyst is detected based on the NOx storage capacity thus estimated (for example, refer to a first patent document).

However, in cases where lower HC such as gasoline is supplied as the reducing agent, the deterioration of the NOx catalyst can be detected according to the above-mentioned technique, but in cases where higher HC such as light oil is supplied as the reducing agent, a detected value of the air fuel ratio sensor may deviate from its actual value, and hence, there will be a fear that the detection accuracy of the deterioration of the NOx catalyst may become low.

### [PRIOR ART REFERENCES]

EP 2119882

### [PATENT DOCUMENTS]

[First Patent Document] Japanese patent application laid-open No. 2000-034946

### [SUMMARY OF THE INVENTION]

### [PROBLEMS TO BE SOLVED BY THE INVENTION]

The present invention has been made in view of the problem as referred to above, and has for its object to provide a technique of performing the determination of deterioration of a NOx storage reduction catalyst in a more accurate manner.

### [MEANS FOR SOLVING THE PROBLEMS]

In order to achieve the above-mentioned object, a catalyst deterioration determination system according to the present invention resides in a catalyst deterioration determination system which determines a deterioration of a NOx storage reduction catalyst which is arranged in an exhaust passage of an internal combustion engine in such a manner that it stores NOx and reduces the NOx stored therein by the supply of a reducing agent, said system comprising:
a supply device that supplies the reducing agent to said NOx storage reduction catalyst thereby to change an air fuel ratio of an exhaust gas passing through said NOx storage reduction catalyst;
a detection device that detects NH₃ in the exhaust gas at the downstream side of said NOx storage reduction catalyst;
a control device that performs a first supply of the reducing agent and a second supply of the reducing agent in a sequential manner by adjusting an amount of the reducing agent in such a manner that the air fuel ratio of the exhaust gas becomes a rich air fuel ratio at the time of supplying the reducing agent from said supply device; and
a determination device that determines based on the detected value of said detection device whether said NOx storage reduction catalyst has deteriorated, after a predetermined period of time has elapsed from the start of said first supply of the reducing agent, and after the start of said second supply of the reducing agent.

The NOx storage reduction catalyst stores NOx when the air fuel ratio of the exhaust gas is lean, and reduces the NOx having been stored therein when the reducing agent exists. The supply device can supply the reducing agent to the NOx storage reduction catalyst. The reducing agent may be supplied into the exhaust gas flowing through the exhaust passage, or may be caused to discharge from the internal combustion engine. Then, the air fuel ratio of the exhaust gas is made lower by supplying the reducing agent.

Here, when the reducing agent is supplied to the NOx storage reduction catalyst thereby to provide a rich air fuel ratio, NOx released from the catalyst and NOx in the exhaust gas flowing into the catalyst react with H₂ and HC thereby to generate NH₃. In the case where the NOx storage reduction catalyst is a new article, the reduction ability thereof is high, so at the beginning of starting the supply of the reducing agent, the NOx stored in the NOx storage reduction catalyst is reduced to N₂, and hence an amount of generation of NH₃ is small. On the other hand, as the deterioration of the NOx storage reduction catalyst progresses, the reduction ability thereof to N₂ decreases, so the amount of generation of NH₃ increases.

Then, when the deterioration of the NOx storage reduction catalyst further progresses, the NOx reduction ability thereof decreases to a remarkable extent, so the amount of generation of NH₃ also decreases. In that case, a difference in the amount of generation of NH₃ between when the NOx storage reduction catalyst is new and when it has deteriorated becomes small, so that it becomes difficult to make the deterioration determination based on the amount of generation of NH₃.

However, when the reducing agent is further supplied to provide a rich fuel ratio after the NOx stored in the NOx storage reduction catalyst has been reduced to N₂, there will be a difference in the amount of generation of NH₃ between when the NOx storage reduction catalyst is new and when it has deteriorated.

That is, when the reducing agent is supplied in a state where the NOx has not been stored in the NOx storage reduction catalyst, a surplus of the reducing agent will occur. This reducing agent reacts with NOx in the exhaust gas to generate NH₃. In the case of the catalyst being new, a large amount of NH₃ will be generated, but in the case of the catalyst having deteriorated, it becomes difficult for the reducing agent and NOx to react with each other, so the amount of generation of NH₃ becomes small.

Therefore, after the NOx stored in the NOx storage reduction catalyst has been reduced by the first supply of the reducing agent, it is possible to determine the deterioration of the NOx storage reduction catalyst on the basis of the detected value of the detection device at the time of the second supply of the reducing agent. That is, the first supply of the reducing agent is carried out in order to decrease the amount of NOx stored in the NOx storage reduction catalyst. On the other hand, the second supply of the reducing agent is carried out mainly for the purpose of causing the NOx in the exhaust gas and the reducing agent to react with each other thereby to generate NH₃.

Here, note that the predetermined period of time can be set as a period of time which is taken for the amount of NH₃ generated when the reducing agent is supplied to reach a value corresponding to the extent of the deterioration of the catalyst. In addition, the predetermined period of time may also be set as a period of time in which the amounts of NH₃ generated by the new catalyst and by the normal catalyst, respectively, become substantially the same level. Moreover, note that at the time of the start of the second supply of the reducing agent, NOx may not have been stored in the NOx storage reduction catalyst, but instead, a certain amount of NOx may also have been stored therein. That is, the NOx stored in the NOx storage reduction catalyst is reduced also by the second supply of the reducing agent, and hence, when the NOx storage reduction catalyst becomes a state in which NOx is not stored therein after the start of the second supply of the reducing agent, NH₃ will be generated thereafter, even in the case of the new catalyst.

Here, note that in the present invention, said control device can adjust the amount of reducing agent in such a manner that the air fuel ratio of the exhaust gas at the time of said second supply of the reducing agent becomes leaner than at the time of said first supply of the reducing agent and richer than a stoichiometric air fuel ratio.

That is, because the degree of richness becomes larger at the time of the first supply of the reducing agent than at the time of the second supply of the reducing agent, it is possible to decrease the amount of the NOx stored in the NOx storage reduction catalyst in a quick manner. As a result, it is possible to quickly determine the deterioration of the NOx storage reduction catalyst. Moreover, at the time of the second supply of the reducing agent, NH₃ can be generated due to the rich air fuel ratio of the exhaust gas, but the amount of consumption of the reducing agent can be reduced by decreasing the degree of richness. Here, note that at the time of the second supply of the reducing agent, a necessary minimum amount of reducing agent only sufficient to react with the NOx in the exhaust gas may be supplied.

In addition, in the present invention, said control device supplies the reducing agent a plurality of times at the time of the second supply of the reducing agent so that the air fuel ratio of the exhaust gas is caused to vary between a rich state and a lean state, and makes a period of time in which the air fuel ratio of the exhaust gas becomes a rich air fuel ratio per one time at the time of said second supply of the reducing agent shorter than a period of time in which the air fuel ratio of the exhaust gas becomes a rich air fuel ratio at the time of said first supply of the reducing agent.

That is, the amount of the NOx stored in the NOx storage reduction catalyst can be decreased at the time of the first supply of the reducing agent. Even if NOx remains in the NOx catalyst at the time of the end of the first supply of the reducing agent, the amount of NOx stored in the NOx catalyst becomes zero by repeating a lean state and a rich state at the time of the second supply of the reducing agent. After that, with the new NOx catalyst, the reducing agent and NOx flowing into the NOx catalyst react with each other thereby to generate NH₃. That is, the amount of generation of NH₃ at the time of the supply of the reducing agent when a certain period of time has passed after the first supply of the reducing agent is started is comparatively large in the case of the normal NOx catalyst including the new NOx catalyst, but is comparatively small in the case of the deteriorated NOx catalyst. Accordingly, the deterioration of the NOx storage reduction catalyst can be determined on the basis of the detected value of the detection device at this time.

In the present invention, when the detected value of said detection device is equal to or more than a threshold value, said determination device can determine that said NOx storage reduction catalyst is normal.

Moreover, in the present invention, when the detected value of said detection device is less than the threshold value, said determination device can determine that said NOx storage reduction catalyst has deteriorated.

At the time of supplying the reducing agent after the certain period of time has passed from the start of the first supply of the reducing agent, in the normal NOx catalyst including the new NOx catalyst, the amount of generation of NH₃ becomes comparatively large, whereas in the deterioration NOx catalyst, the amount of generation of NH₃ becomes comparatively small. In the normal NOx catalyst, by setting as the threshold value a lower limit value of the detected value detected by the detection device, it is possible to determine whether the NOx catalyst is normal or has deteriorated.

In the present invention, said detection device may be a NOx sensor that detects NOx and NH₃ in the exhaust gas.

The NOx sensor detects NH₃ as well as NOx. Therefore, it is impossible to distinguish whether the detected value of the NOx sensor is a concentration of NOx or a concentration of NH₃. However, when the reducing agent is supplied until the air fuel ratio of the exhaust gas becomes a rich air fuel ratio, almost no NOx will be contained in the exhaust gas at the downstream side of the NOx storage reduction catalyst. For this reason, the detected value of the NOx sensor will indicate the concentration of NH₃. Accordingly, NH₃ can be detected by the use of the NOx sensor. As a result, the number of sensors to be installed can be decreased, thus making it possible to suppress an increase in the cost.

### [EFFECT OF THE INVENTION]

According to the present invention, it is possible to carry out the determination of deterioration of a NOx storage reduction catalyst in a more accurate manner.

### [BRIEF DESCRIPTION OF THE DRAWINGS]

[Fig. 1] is a view showing the schematic construction of an internal combustion engine and its exhaust system according to embodiments of the present invention.
[Fig. 2] is a view for explaining a NOx storage or adsorption function in a NOx catalyst.
[Fig. 3] is a view for explaining a NOx reduction function in the NOx catalyst.
[Fig. 4] is a view showing the relation between an air fuel ratio and a concentration of NH₃ at the downstream side of the NOx catalyst, at the time of supplying a reducing agent.
[Fig. 5] is a view showing the relation between an air fuel ratio and a concentration of NH₃ at the downstream side of the NOx catalyst, at the time of rich spike control according to a first embodiment of the present invention.
[Fig. 6] is a flow chart showing a flow for the determination of deterioration of the NOx catalyst according to the first embodiment of the present invention.
[Fig. 7] is a view showing the relation between an air fuel ratio and a concentration of NH₃ at the downstream side of the NOx catalyst, at the time of rich spike control according to a second embodiment of the present invention.
[Fig. 8] is a flow chart showing a flow for the determination of deterioration of the NOx catalyst according to the second embodiment of the present invention.

### [MODES FOR CARRYING OUT THE INVENTION]

Hereinafter, reference will be made to specific embodiments of a catalyst deterioration determination system according to the present invention based on the attached drawings.

### [First Embodiment]

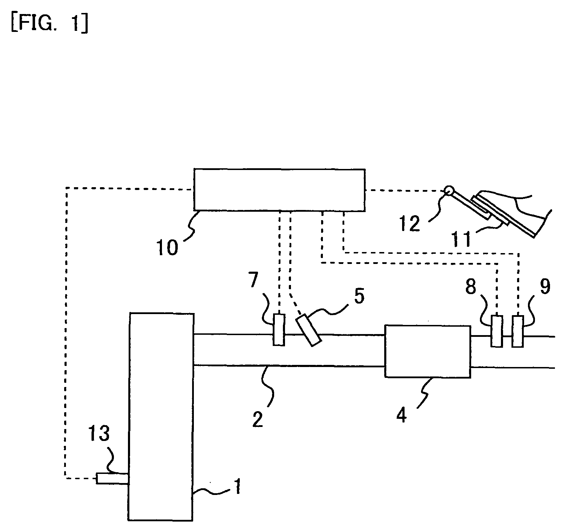

Fig. 1 is a view showing the schematic construction of an internal combustion engine and its exhaust system according to this first embodiment of the present invention. The internal combustion engine 1 shown in Fig. 1 is a four-cycle diesel engine of a water cooled type having four cylinders.

An exhaust passage 2 is connected to the internal combustion engine 1. In the middle of this exhaust passage 2, there is arranged a NOx storage reduction catalyst 4 (hereinafter, referred to as a NOx catalyst 4).

The NOx catalyst 4 is constructed such that alumina (Al₂O₃), for example, is used as a carrier, and barium (Ba) and platinum (Pt), for example, are carried on the carrier.

This NOx catalyst 4 has a function of occluding or storing NOx contained in an incoming exhaust gas when the oxygen concentration of the exhaust gas is high, and of reducing the occluded or stored NOx when the oxygen concentration of the incoming exhaust gas becomes low and when a reducing agent exists.

In addition, an injection valve 5, which serves to inject the reducing agent into the exhaust gas, is mounted on the exhaust passage 2 at the upstream side of the NOx catalyst 4. The injection valve 5 is opened by means of a signal from an ECU 10 which will be described later, so that it injects the reducing agent into the exhaust gas. For the reducing agent, there is used, for example, fuel (light oil) in the internal combustion engine 1, but it is not limited to this.

The fuel injected from the injection valve 5 into the exhaust passage 2 decreases the air fuel ratio of the exhaust gas which has flowed from an upstream side of the exhaust passage 2. Then, at the time of reducing the NOx stored in the NOx catalyst 4, so-called rich spike control is carried out in which the air fuel ratio of the exhaust gas flowing into the NOx catalyst 4 is caused to decrease in a relatively short period or cycle by injecting fuel from the injection valve 5. The amount of the reducing agent to be injected from the injection valve 5 is decided based, for example, on an operating state (the number of engine revolutions per unit time and the amount of fuel injection) of the internal combustion engine 1. The relation among the amount of reducing agent, the number of engine revolutions per unit time and the engine load can be mapped in advance. In addition, an air fuel ratio sensor may be mounted on the exhaust passage 2, and feedback control may be performed on the amount of the reducing agent in such a manner that the air fuel ratio detected by the air fuel ratio sensor becomes a target value.

Here, note that in this embodiment, the injection valve 5 corresponds to a supply device in the present invention. In addition, the reducing agent can also be supplied by causing unburnt fuel to be discharged or delivered from the internal combustion engine 1. That is, provision can be made for a cylinder injection valve which serves to inject fuel into a cylinder, so that a gas containing a large amount of reducing agent can also be caused to discharge or deliver from the internal combustion engine 1, by performing auxiliary injection (post injection) in which fuel is once again injected during an expansion stroke or an exhaust stroke after main injection has been carried out from the cylinder injection valve, or by retarding the timing of fuel injection from the cylinder injection valve.

In addition, an upstream side NOx sensor 7 for measuring the concentration of NOx in the exhaust gas is mounted on the exhaust passage 2 at a location upstream of the injection valve 5. Also, a downstream side NOx sensor 8 for measuring the concentration of NOx in the exhaust gas and a temperature sensor 9 for measuring the temperature of the exhaust gas are mounted on the exhaust passage 2 at locations downstream of the NOx catalyst 4. Here, note that in this embodiment, the downstream side NOx sensor 8 corresponds to a detection device or a NOx sensor in the present invention.

In the internal combustion engine 1 constructed as mentioned above, there is arranged in combination therewith the ECU 10 which is an electronic control unit for controlling the internal combustion engine 1. This ECU 10 controls the operating state of the internal combustion engine 1 in accordance with the operating conditions of the internal combustion engine 1 and/ or driver's requirements.

Further, besides the above-mentioned sensors, an accelerator opening sensor 12, which serves to detect the engine load by outputting an electrical signal corresponding to an amount by which a driver depressed an accelerator pedal 11, and a crank position sensor 13, which serves to detect the number of revolutions per minute of the engine, are connected to the ECU 10 through electrical wiring, and the output signals of these variety of kinds of sensors are inputted to the ECU 10.

On the other hand, the injection valve 5 is connected to the ECU 10 through electrical wiring, so that the opening and closing timing of the injection valve 5 is controlled by the ECU 10. Here, note that in this embodiment, the ECU 10, which adjusts the amount of the reducing agent to be supplied from the injection valve 5, corresponds to a control device in the present invention.

Then, the ECU 10 carries out first rich spike control (a first supply of the reducing agent) for the purpose of adjusting the air fuel ratio of the exhaust gas to be a first rich air fuel ratio (e.g., the air fuel ratio being less than 13). This first rich spike control is carried out in order to decrease the amount of NOx stored in the NOx catalyst 4. Accordingly, the first rich air fuel ratio may also be set as an air fuel ratio which is suitable for quickly decreasing the NOx stored in the NOx catalyst 4.

Immediately after the first rich spike control has been completed, second rich spike control (a second supply of the reducing agent) is carried out for the purpose of adjusting the air fuel ratio of the exhaust gas to be a second rich air fuel ratio (the air fuel ratio being more than or equal to 13 and less than 14, for example) in such a manner that it is leaner than the first rich air fuel ratio and is richer than a stoichiometric air fuel ratio. This second rich spike control is carried out in order to cause the NOx in the exhaust gas to react with the reducing agent to generate NH₃. Accordingly, the second rich air fuel ratio may also be set as an air fuel ratio which is suitable for generating NH₃. In addition, a necessary minimum amount of reducing agent may be supplied so as to generate NH₃.

Then, after the second rich spike control has been started, and after a predetermined period of time has elapsed from the start of the first rich spike control, the determination of deterioration of the NOx catalyst 4 is carried out based on the concentration of NH₃ detected by the downstream side NOx sensor 8. The predetermined period of time is set as a period of time until the amount of NH₃ generated when the reducing agent is supplied becomes an amount corresponding to the extent of the deterioration of the NOx catalyst 4. In addition, the predetermined period of time may also be set as a period of time required for the amount of NOx stored in the NOx catalyst 4 to decrease to a sufficient extent, and it may also be set, for example, as a period of time until the amount of NOx stored in the NOx catalyst 4 becomes equal to or less than a predetermined amount. Here, note that the second rich spike control can also be started after the predetermined period of time has elapsed from the start of the first rich spike control.

Here, note that in the downstream side NOx sensor 8, NH₃ reacts with O₂ to form NO, so that it is detected as NOx. For this reason, it is difficult to determine whether NH₃ has been detected or NOx has been detected, by the downstream side NOx sensor 8. However, by making the air fuel ratio of the exhaust gas to be a rich air fuel ratio, almost no NOx will be included in the exhaust gas flowing out of the NOx catalyst 4. That is, when the air fuel ratio of the exhaust gas is a rich air fuel ratio, the reducing agent (H₂, HC) and NO react with each other in the NOx catalyst 4 to generate NH₃, so most of what is detected by the downstream side NOx sensor 8 becomes NH₃. Accordingly, what is detected by the downstream side NOx sensor 8 at this time will be NH₃.

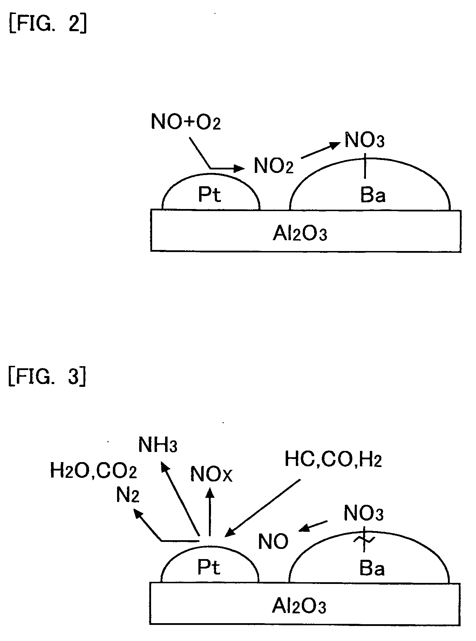

Here, Fig. 2 is a view for explaining a NOx storage or adsorption function in the NOx catalyst 4. Also, Fig. 3 is a view for explaining a NOx reduction function in the NOx catalyst 4.

When the air fuel ratio of the exhaust gas is lean, the NOx catalyst 4 functions to oxidize NO with O₂ on Pt to form NO₃, and to cause Ba to adsorb or store it as Ba(NO₃)₂ (refer to Fig. 2). On the other hand, when the reducing agent is supplied to make the air fuel ratio of the exhaust gas rich, Ba(NO₃)₂ will be reduced to NO₂, which will be released and further reduced to N₂ on Pt (refer to Fig. 3).

However, when the NOx catalyst 4 deteriorates (causes sintering), the surface area of Pt will become smaller than at the normal time. For this reason, the released NO₂ becomes difficult to be reduced to N₂. When the air fuel ratio of the exhaust gas is made into a rich air fuel ratio, the NOx desorbed or separated from the NOx catalyst 4 or the NOx in the exhaust gas flowing into the NOx catalyst 4 reacts with the reducing agent (H₂ or HC) in the NOx catalyst 4, so that NH₃ is thereby generated. At the time of a rich air fuel ratio, whether in cases where the NOx catalyst 4 is normal or deteriorated, H₂ or HC reacts with NO to generate NH₃ in the NOx catalyst 4.

However, as the NOx catalyst 4 deteriorates, the amount of Ba able to occlude or store NOx is decreased due to sintering of Pt. For this reason, the occlusion or storage capacity of the NOx catalyst 4 decreases. That is, as the NOx catalyst 4 deteriorates, the amount of NOx stored as Ba(NO₃)₂ will decrease. Then, in accordance with the decreasing surface area of Pt, the amount of NH₃ generated at the time of supplying the reducing agent also decreases.

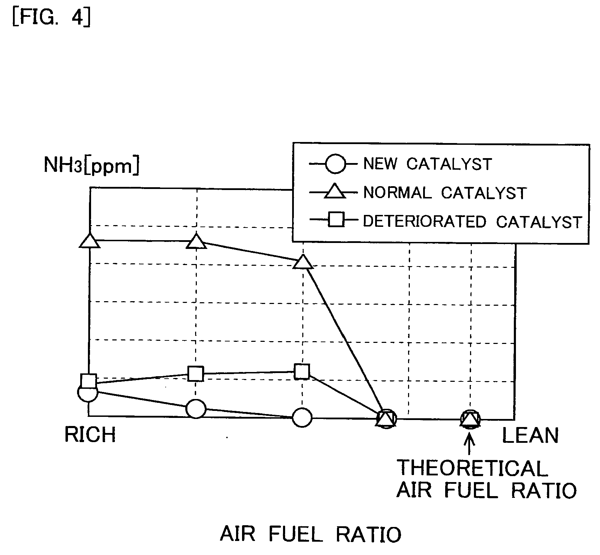

Here, Fig. 4 is a view showing the relation between the air fuel ratio and the concentration of NH₃ at the downstream side of the NOx catalyst 4 at the time of supplying the reducing agent. The concentration of NH₃ is a concentration at a predetermined time after the start of the supply of the reducing agent until all the NOx stored in the NOx catalyst 4 has been desorbed or released. A "new catalyst" indicates the NOx catalyst 4 which has just been fitted or installed on a vehicle. This is in a state in which the mileage of the vehicle is in the range of 0 to several km, with almost no deterioration of Pt. A "normal catalyst" indicates the NOx catalyst 4 in which Pt has deteriorated to a certain extent, but the extent of the deterioration thereof is within an allowable range. A "deteriorated catalyst" indicates the NOx catalyst 4 in which the extent of the deterioration thereof has exceeded the allowable range.

As shown in Fig. 4, in the rich air fuel ratio, the concentration of NH₃ in the new catalyst is the lowest, and the concentration of NH₃ in the normal catalyst is the highest. Here, with the new catalyst, the reducing ability thereof is high, and hence, when the reducing agent is supplied, most of the NOx is reduced to N₂. For this reason, the amount of NH₃ to be generated becomes small. In addition, as the deterioration of the NOx catalyst 4 progresses, the ability or capacity of the NOx catalyst 4 to reduce NOx to N₂ decreases, so the amount of NH₃ to be generated increases. However, as the deterioration of the NOx catalyst 4 further progresses, the NOx reduction ability thereof will remarkably decrease, and the amount of NH₃ to be generated will also reduce. For this reason, the concentration of NH₃ may be the same in the normal catalyst and in the deteriorated catalyst, and so, it is difficult to carry out the determination of deterioration of the NOx catalyst 4 based on the detected value of the downstream side NOx sensor 8 at the time of reducing the NOx stored in the NOx catalyst 4.

However, when the reducing agent is supplied to generate NH₃ in a state where NOx is not stored in the NOx catalyst 4, and the amount of NH₃ to be generated at this time will change according to the extent of the deterioration of the NOx catalyst 4. That is, based on the detected value of the downstream side NOx sensor 8 at this time, it is possible to carry out the determination of the deterioration of the NOx catalyst 4.

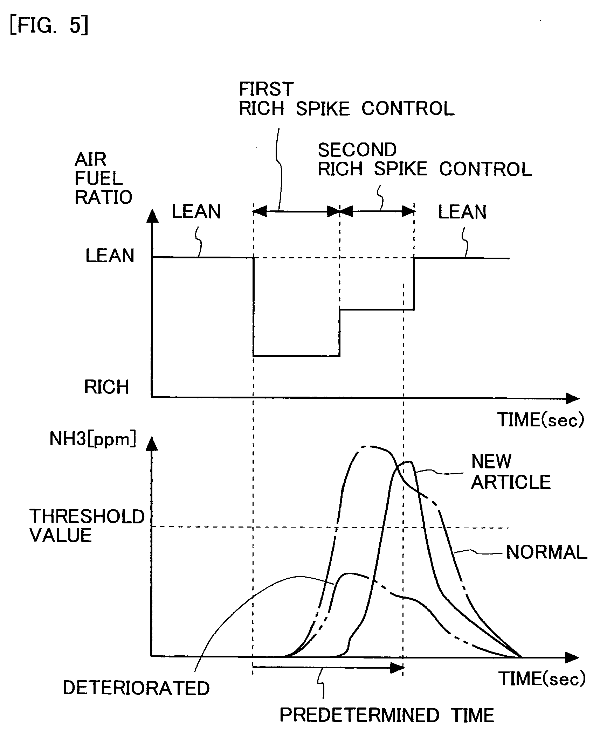

Fig. 5 is a view showing the relation between the air fuel ratio of the exhaust gas and the concentration of NH₃ at the downstream side of the NOx catalyst 4 at the time of the rich spike control according to this embodiment. In the concentration of NH₃, a solid line indicates the case of the above-mentioned new catalyst, a dashed line indicates the case of the above-mentioned normal catalyst, and an alternate long and two short dashes line indicates the case of the above-mentioned deteriorated catalyst. After the times of before and after supplying the reducing agent, the air fuel ratio of the exhaust gas is a lean air fuel ratio. In addition, in the first rich spike control, the air fuel ratio is set to be less than 13, for instance, and in the second rich spike control, the air fuel ratio is made higher than in the first rich spike control, and to be more than or equal to 13 and less than 14, for instance.

This first rich spike control is to decrease the amount of NOx stored in the NOx catalyst 4. In the first rich spike control, the air fuel ratio is relatively low, so it is possible to reduce the NOx stored in the NOx catalyst 4 to N₂ in a quick manner. That is, NO reacts with CO, H₂ or HC so that it is reduced to N₂.

Here, in the case where the NOx catalyst 4 is the above-mentioned new catalyst, Pt therein has not deteriorated, so reduction reaction becomes active, and most of the NOx stored in the NOx catalyst 4 is reduced to N₂, as a result of which the amount of generation of NH₃ is small, and the concentration of NH₃ becomes low. On the other hand, in the case of the normal catalyst and the deteriorated catalyst, the deterioration of Pt progresses, and hence the reduction ability of the NOx catalyst 4 from NOx to N₂ decreases, so that the amount of generation of NH₃ increases, and the concentration of NH₃ becomes high.

The second rich spike control is to generate NH₃. When the reducing agent is further supplied after the reduction of the NOx stored in the NOx catalyst 4 has been completed, NO contained in the exhaust gas flowing into the NOx catalyst 4 reacts with H₂ or HC, thereby generating NH₃.

2NO + 5H₂→ 2NH₃ + 2H₂O

NO + HC + H₂O → NH₃ + CO₂

Here, note that a necessary minimum amount of reducing agent may be supplied so as to generate NH₃. For example, the necessary minimum amount of the reducing agent may be supplied according to the concentration of NOx detected by the upstream side NOx sensor 7.

Here, in the case where the NOx catalyst 4 is the above-mentioned new catalyst, NOx will not be stored in the said NOx catalyst 4 by carrying out the first rich spike control. In addition, because the new catalyst has a high reduction ability, at the time of the second rich spike control, the NOx in the exhaust gas reacts with the reducing agent to generate a large amount of NH₃. In the case where the NOx catalyst 4 is the above-mentioned normal catalyst, Pt therein has deteriorated, but the NOx in the exhaust gas reacts with the reducing agent to generate a lot of NH₃. On the other hand, in the case where the NOx catalyst 4 is the above-mentioned deteriorated catalyst, the reduction ability of the NOx catalyst 4 becomes low, and hence the amount of generation of NH₃ becomes small. Accordingly, the amount of NH₃ flowing out downwardly of this NOx catalyst 4 decreases according to the extent of the deterioration of the NOx catalyst 4.

In that case, at the time of the first rich spike control, the amount of generation of NH₃ in the new catalyst is small, so it was difficult to make the determination of deterioration of the NOx catalyst 4 on the basis of the concentration of NH₃, but at the time of the second rich spike control, the amount of generation of NH₃ in the new catalyst increases, so that it becomes possible to make the determination of deterioration of the NOx catalyst 4 on the basis of the concentration of NH₃.

That is, after a predetermined time has passed from a time point at which the first rich spike control has been started, the determination of deterioration of the NOx catalyst 4 can be made on the basis of the concentration of NH₃. Then, the determination can be made as follows: when the concentration of NH₃ is equal to or greater than a threshold value, the NOx catalyst 4 is normal, whereas when it is less than the threshold value, the NOx catalyst 4 has deteriorated. Thus, if the concentration of NH₃ that flows out from NOx catalyst 4 is detected with downstream side NOx sensor 8, it will be possible to make the deterioration of NOx catalyst 4.

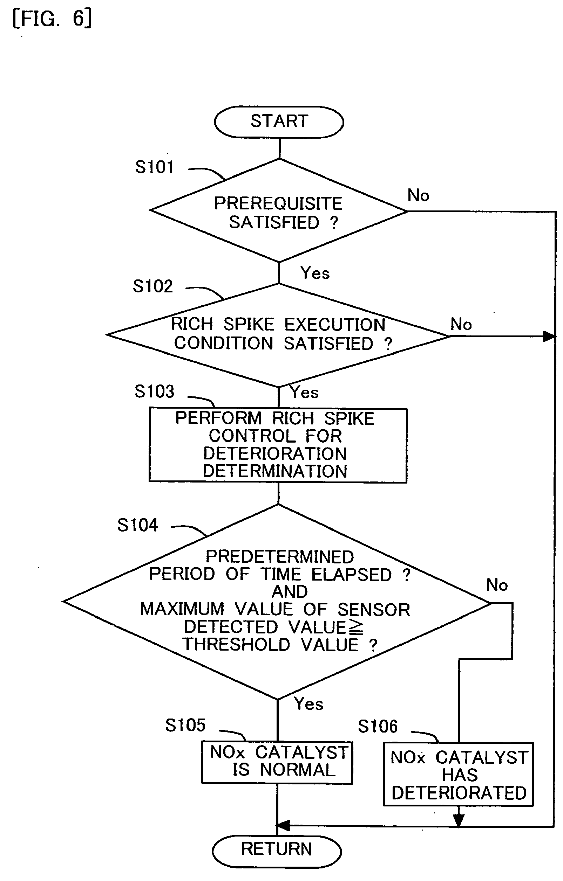

Fig. 6 is a flow chart showing a flow for the determination of deterioration of the NOx catalyst 4 according to this embodiment. This routine is carried out at each predetermined period of time.

In step S101, it is determined whether a prerequisite or precondition for performing the determination of deterioration of the NOx catalyst 4 is satisfied. For example, when the downstream side NOx sensor 8 is normal and when the temperature of the NOx catalyst 4 is a temperature suitable for the reduction of NOx, a determination is made that the prerequisite is satisfied. Whether the downstream side NOx sensor 8 is normal or not can be determined by means of well-known techniques. In addition, the temperature suitable for the reduction of NOx is a temperature at the time when the NOx catalyst 4 has been activated, for example. The temperature of the NOx catalyst 4 is detected by the temperature sensor 9.

In cases where an affirmative determination is made in step S101, the routine advances to step S102, whereas in cases where a negative determination is made, this routine is ended.

In step S102, it is determined whether a condition for executing the rich spike control (hereinafter referred to as rich spike execution condition) is satisfied. The rich spike execution condition is a condition under which rich spike control is executed for carrying out the determination of deterioration of the NOx catalyst 4. For example, when an amount of NOx equal to or more than the predetermined amount is stored in the NOx catalyst 4, a determination is made that the rich spike execution condition is satisfied. The amount of NOx stored in the NOx catalyst 4 is calculated based on the concentration of NOx detected by the upstream side NOx sensor 7. The predetermined amount referred to herein has been obtained through experiments, etc., in advance as a value at which the reducing agent, when supplied, can be consumed. That is, if NOx has not been stored in the NOx catalyst 4, the reducing agent will be excessively supplied at the time of the first rich spike control, so that the reducing agent may pass sideways through the NOx catalyst 4. Thus, in such a case, the determination of deterioration is not carried out.

In cases where an affirmative determination is made in step S102, the routine goes to step S103, whereas in cases where a negative determination is made, this routine is ended.

In step S103, rich spike control for the determination of deterioration of the NOx catalyst 4 is carried out. That is, the first rich spike control, in which the degree of richness is relatively large, and the second rich spike control, in which the degree of richness is relatively small, are carried out continuously.

In step S104, it is determined whether the predetermined period of time has elapsed from the start of the rich spike control in step S103, and whether a maximum value of the detected value of the downstream side NOx sensor 8 is equal to or larger than the threshold value. This predetermined period of time is a period of time required for the amount of NH₃ generated by the reaction of the NOx in the exhaust gas and the reducing agent after the reduction of NOx stored in the NOx catalyst 4 has been completed to become an amount corresponding to the extent of the deterioration of the NOx catalyst 4. Here, note that an optimum value for this predetermined period of time may have beforehand been obtained through experiments, etc. The second rich spike control is caused to continue until the predetermined period of time elapses from the start of the first rich spike control. In addition, the threshold value is a detected value which becomes a boundary of whether the NOx catalyst 4 has deteriorated or not, and it has been set in advance.

In cases where an affirmative determination is made in step S104, the routine advances to step S105, in which a determination is made that the NOx catalyst 4 is normal. On the other hand, in cases where a negative determination is made in step S104, the routine advances to step S106, in which a determination is made that the NOx catalyst 4 has deteriorated. Here, note that in this embodiment, the ECU 10, which carries out the processing of step S103 through step S106, corresponds to a determination device in the present invention.

As described above, according to this first embodiment, it is possible to carry out the determination of deterioration of the NOx catalyst 4 based on the concentration of NH₃ after reducing the NOx stored in the NOx catalyst 4. In addition, the NOx stored in the NOx catalyst 4 can be quickly decreased by making the air fuel ratio lower in the first rich spike control. As a result, the determination of deterioration of the NOx catalyst 4 can be carried out in a quick manner. Moreover, by relatively decreasing the amount of supply of the reducing agent in the second rich spike control, it is possible to suppress a large amount of NH₃ from being generated, thus making it possible to suppress the NH₃ from being released into the atmospheric air. Also, it is possible to decrease the amount of consumption of the reducing agent.

### [Second Embodiment]

In this second embodiment, first of all, the first rich spike control to reduce the NOx stored in the NOx catalyst 4 is carried out. Then, the second rich spike control to generate NH₃ is carried out. Here, note that in the first rich spike control and the second rich spike control, air fuel ratios to be set as targets, respectively, may be the same. In addition, in the second rich spike control, the reducing agent is supplied in a plurality of times so that the air fuel ratio of the exhaust is caused to change alternately between a rich one and a lean one in a repeated manner. Moreover, in the second rich spike control, a period of time per spike in which the air fuel ratio is put into a rich air fuel ratio is made shorter than that at the time of the first rich spike control. Then, the determination of deterioration of the NOx catalyst 4 is carried out based on the concentration of NH₃ during the execution of the second rich spike control. The other devices, parts and so on are the same as those in the first embodiment, so the explanation thereof is omitted.

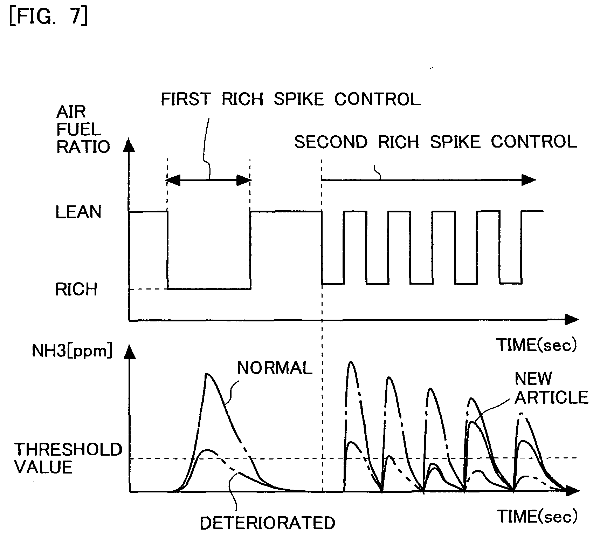

Fig. 7 is a view showing the relation between the air fuel ratio of the exhaust gas and the concentration of NH₃ at the downstream side of the NOx catalyst 4, at the time of the rich spike control according to this second embodiment. In the concentration of NH₃, a solid line indicates the case of the above-mentioned new catalyst, an alternate long and short dash line indicates the case of the above-mentioned normal catalyst, and an alternate long and two short dashes line indicates the case of the above-mentioned deteriorated catalyst. At the times of before and after supplying the reducing agent, the air fuel ratio of the exhaust gas is a lean air fuel ratio. In addition, in the first rich spike control, ordinary control for reducing the NOx stored in the NOx catalyst 4 is carried out. At this time, the reducing agent is supplied in such a manner that the air fuel ratio of the exhaust gas becomes less than 14, for example.

On the other hand, in the second rich spike control, the air fuel ratio of the exhaust gas may be the same as in the first rich spike control. That is, the reducing agent is supplied in such a manner that the air fuel ratio of the exhaust gas becomes less than 14, for example.

The first rich spike control is mainly for decreasing the NOx stored in the NOx catalyst 4. Here, in the case where the NOx catalyst 4 is the above-mentioned new catalyst, Pt therein has not deteriorated, so reduction reaction becomes active, and most of the NOx stored in the NOx catalyst 4 is reduced to N₂. As a result, the concentration of NH₃ becomes low. On the other hand, in the case of the normal catalyst and the deteriorated catalyst, the deterioration of Pt has progressed, so that the reduction ability of the NOx catalyst 4 from NOx to N₂ decreases. As a result, the reducing agent becomes an excessive state, and so, the concentration of NH₃ becomes high. And, there may be a small difference in the concentration of NH₃ detected at the time of the first rich spike control between the new catalyst and the deteriorated catalyst or between the normal catalyst and the deteriorated catalyst. Accordingly, the determination of deterioration of the NOx catalyst 4 is not carried out by using the concentration of NH₃ detected at the time of the first rich spike control. Here, note that the first rich spike control can be ordinary rich spike control for reducing NOx which is also carried out when the determination of deterioration of the NOx catalyst 4 is not carried out.

Here, note that in Fig. 7, from the end of the first rich spike control until the start of the second rich spike control, there exists a period of time in which the reducing agent is not supplied, and hence, during this period of time, NOx is stored by the NOx catalyst 4. In addition, NOx may remain in the NOx catalyst 4 at the time of the end of the first rich spike control. For this reason, with the new catalyst, NH₃ is rarely detected at the time of the start of the second rich spike control.

The second rich spike control is mainly for generating NH₃. When the reducing agent is further supplied after carrying out the first rich spike control, the NOx catalyst 4 will be in a state where NOx has not been stored therein. Then, when the reducing agent is supplied further, the NO contained in the exhaust gas flowing into the NOx catalyst 4 will react with H₂ or HC, thereby generating NH₃.

Here, in the case where the NOx catalyst 4 is the above-mentioned normal catalyst, Pt therein has deteriorated to a certain extent, but the NOx in the exhaust gas reacts with the reducing agent, so that a lot of NH₃ is generated. On the other hand, in the case where the NOx catalyst 4 is the above-mentioned deteriorated catalyst, the reduction ability thereof becomes low due to the decreased surface area of Pt, so that the amount of NH₃ to be generated decreases.

In addition, in the case where the NOx catalyst 4 is the new catalyst, when the NOx stored in the NOx catalyst 4 is exhausted, the reducing agent becomes an excessive state. This reducing agent reacts with NOx freshly flowing into the NOx catalyst 4, so that NH₃ is thereby generated. In the second rich spike control, the supply of the reducing agent is carried out a plurality of times, and hence, after the NOx stored in the NOx catalyst 4 has been exhausted, NH₃ is generated each time the reducing agent is supplied to the new catalyst. Then, when the supply of the reducing agent is carried out in a repeated manner, the amount of NH₃ flowing to the downstream side of the NOx catalyst 4 will be reversed between the new catalyst and the deteriorated catalyst.

In that case, at the time of the first rich spike control, it is difficult to carry out the determination of deterioration of the NOx catalyst 4 based on the concentration of NH₃, but at the time of the second rich spike control, the amount of NH₃ generated in the new catalyst increases, so that it becomes possible to carry out the determination of deterioration of the NOx catalyst 4 based on the concentration of NH₃.

For example, when the maximum value of the concentration of NH₃ detected by the downstream side NOx sensor 8 at the time of the execution of the second spike control is equal to or greater than the threshold value, the NOx catalyst 4 can be determined to be normal, whereas when it is less than the threshold value, the NOx catalyst 4 can be determined to have deteriorated. The threshold value can be set to the maximum value of the concentration of NH₃ which is detected at the time when the NOx catalyst 4 is on the boundary of whether it is normal or it has deteriorated

Moreover, in the second rich spike control, the air fuel ratio of the exhaust gas is caused to change between a rich state and a lean state in a repeated manner, so that the detection of NH₃ can be carried out a plurality of times. Thus, by detecting NH₃ a plurality of times, it is possible to enhance the accuracy of the determination of deterioration of the NOx catalyst 4.

For instance, after the predetermined time has elapsed from the point in time at which the first rich spike control has been started, the second rich spike control is started, and the local maximum value of the concentration of NH₃ detected by the downstream side NOx sensor 8 is stored each time the reducing agent is supplied at the time of the execution of the second rich spike control, wherein when the number of times (frequency) in which the local maximum value becomes equal to or more than the threshold value is equal to or more than a predetermined number of times, a determination can be made that the NOx catalyst 4 is normal. On the other hand, when the number of times in which the local maximum value of the concentration of NH₃ becomes equal to or greater than the threshold value is less than the predetermined number of times, a determination can be made that the NOx catalyst 4 has deteriorated. The predetermined period of time at this time is a period of time required to recover the occlusion or storage capacity of the NOx catalyst 4, and it may be a period of time required for ordinary rich spike control which is ordinarily carried out for reducing the NOx stored in the NOx catalyst 4.

In this manner, by detecting the concentration of NH₃ flowing out from the NOx catalyst 4 at the time of the second rich spike control by means of the downstream side NOx sensor 8, it is possible to determine the deterioration of the NOx catalyst 4.

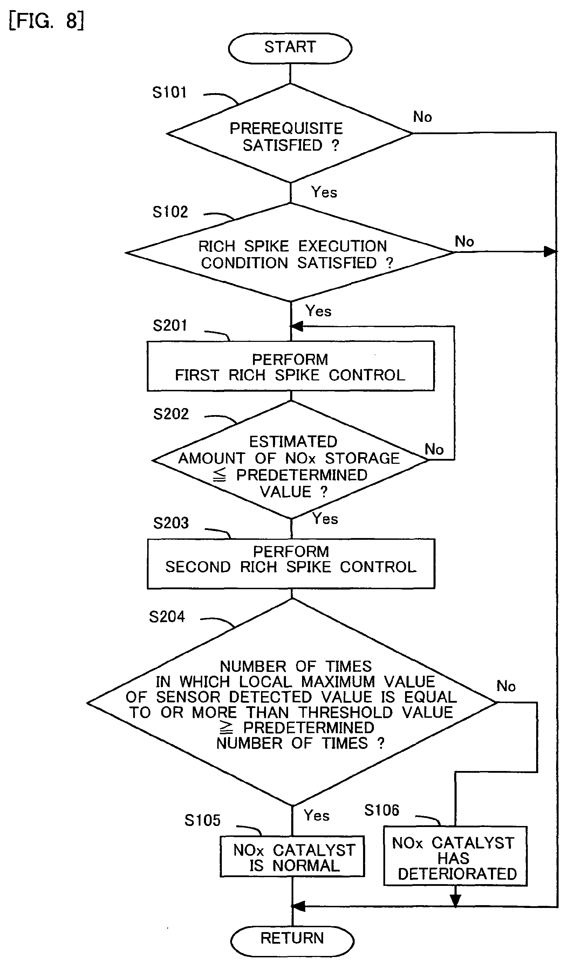

Fig. 8 is a flow chart showing a flow for the determination of deterioration of the NOx catalyst 4 according to this embodiment. This routine is carried out in a repeated manner at each predetermined time interval. Here, note that whole for those steps in which the same processing as in the above-mentioned flow is carried out, the same symbols are attached, and the explanation thereof is omitted.

In step S201, the first rich spike control is executed. In this step, the reducing agent is supplied in order to decrease the amount of the NOx stored in the NOx catalyst 4.

In step S202, it is determined whether an estimated value of the amount of NOx (hereinafter, also referred to as an estimated storage amount of NOx) stored in the NOx catalyst 4 is equal to or less than a predetermined value. In this step, it is determined whether the amount of NOx stored in the NOx catalyst 4 has decreased to a sufficient extent. The actual amount of NOx stored in the NOx catalyst 4 decreases according to the period of time elapsed after the first rich spike control has been started, and hence the relation between the elapsed period of time and the estimated storage amount of NOx has been mapped, and stored in the ECU 10. Here, note that when the period of time elapsed after the first rich spike control has been started is equal to or greater than a predetermined period of time, a determination may be made that the estimated storage amount of NOx is equal to or less than the predetermined value. Moreover, the estimated storage amount of NOx can also be obtained by means of well-known techniques.

In cases where an affirmative determination is made in step S202, the routine goes to step S203, whereas in cases where a negative determination is made, a return to step S201 is carried out.

In step S203, the second rich spike control is executed. That is, the supply of the reducing agent for a period of time shorter than that in the first rich spike control is repeatedly carried out a plurality of times. An optimum value for the number of times to be repeated can be obtained in advance through experiments, etc.

In step S204, it is determined whether the number of times in which the local maximum value of the detected value of the downstream side NOx sensor 8 becomes equal to or greater than the threshold value is equal to or more than the predetermined number of times. The threshold value and the predetermined number of times are values which become boundaries of whether the NOx catalyst 4 has deteriorated, and these values have been obtained in advance through experiments, et al. The local maximum value of the detected value of the downstream side NOx sensor 8 is stored each time the supply of the reducing agent is carried out a plurality of times. Here, note that as the deterioration of the NOx catalyst progresses, a maximum value among the local maximum values detected the plurality of times becomes smaller, and the number of times in which the above-mentioned local maximum value becomes equal to or greater than the threshold value also decreases. For this reason, the above-mentioned maximum value at the time when the NOx catalyst 4 is on the boundary of whether or not it has deteriorated may be set as a threshold value, so that a determination may be made as to whether the maximum value of the detected value of the downstream side NOx sensor 8 is equal to or greater than this threshold value.

In cases where an affirmative determination is made in step S204, the routine advances to step S105, in which a determination is made that the NOx catalyst 4 is normal. On the other hand, in cases where a negative determination is made in step S204, the routine advances to step S106, in which a determination is made that the NOx catalyst 4 has deteriorated. Here, note that in this embodiment, the ECU 10, which carries out the processing of step S201 through step S106, corresponds to the determination device in the present invention.

As described above, according to this second embodiment, it is possible to carry out the determination of deterioration of the NOx catalyst 4 based on the concentration of NH₃ after the NOx stored in the NOx catalyst 4 has been reduced. In addition, by decreasing the NOx stored in the NOx catalyst 4 in the first rich spike control, the determination of deterioration of the NOx catalyst 4 becomes possible in a short time after the start of the second rich spike control. Moreover, by causing the air fuel ratio of the exhaust gas to vary between a rich state and a lean state a plurality of times in the second rich spike control, it is possible to enhance the accuracy of the determination of deterioration of the NOx catalyst 4.

### [EXPLANATION OF REFERENCE NUMERALS AND CHARACTERS]

1 internal combustion engine
2 exhaust passage
4 NOx storage reduction catalyst
5 injection valve
7 upstream side NOx sensor
8 downstream side NOx sensor
9 temperature sensor
10 ECU
11 accelerator pedal
12 accelerator opening sensor
13 crank position sensor

## Claims

1. A catalyst deterioration determination system which determines deterioration of a NOx storage reduction catalyst (4) which is arranged in an exhaust passage of an internal combustion engine in such a manner that it stores NOx and reduces the NOx stored therein by the supply of a reducing agent, said system comprising:
a supply device (5) that supplies the reducing agent to said NOx storage reduction catalyst thereby to change an air fuel ratio of an exhaust gas passing through said NOx storage reduction catalyst;
a detection device (8) comprising means for detecting NH₃ in the exhaust gas at the downstream side of said NOx storage reduction catalyst;
a control device (10) comprising means for performing a first supply of the reducing agent and a second supply of the reducing agent in a sequential manner by adjusting an amount of the reducing agent in such a manner that the air fuel ratio of the exhaust gas becomes a rich air fuel ratio at the time of supplying the reducing agent from said supply device; and
a determination device (10) comprising means for determing based on the detected value of said detection device whether said NOx storage reduction catalyst has deteriorated, after a period of time, which is required for the amount of NH₃ generated by the reaction of the NOx in the exhaust gas and the reducing agent after the reduction of NOx stored in said NOx storage reduction catalyst has been completed to become an amount corresponding to the extent of the deterioration of said NOx storage reduction catalyst, has elapsed from the start of said first supply of the reducing agent, and after the start of said second supply of the reducing agent.

2. The catalyst deterioration determination system as set forth in claim 1, wherein said control device (10) comprinsing means for ajusting the amount of reducing agent in such a manner that the air fuel ratio of the exhaust gas at the time of the second supply of the reducing agent becomes leaner than at the time of the first supply of the reducing agent and richer than a stoichiometric air fuel ratio.

3. The catalyst deterioration determination system as set forth in claim 1, wherein said control device (10) comprising means for supplying the reducing agent a plurality of times at the time of the second supply of the reducing agent so that the air fuel ratio of the exhaust gas is caused to vary between a rich state and a lean state, and makes a period of time in which the air fuel ratio of the exhaust gas becomes a rich air fuel ratio per one time at the time of said second supply of the reducing agent shorter than a period of time in which the air fuel ratio of the exhaust gas becomes a rich air fuel ratio at the time of said first supply of the reducing agent.

4. The catalyst deterioration determination system as set forth in any one of claims 1 through 3, wherein when the detected value of said NH₃ detection device (8) is equal to or greater than a threshold value, said determination device (10) makes a determination that said NOx storage reduction catalyst (4) has deteriorated.

5. The catalyst deterioration determination system as set forth in any one of claims 1 through 3, wherein when the detected value of said detection device (8) is less than a threshold value, said determination device (10) makes a determination that said NOx storage reduction catalyst has deteriorated.

6. The catalyst deterioration determination system as set forth in any one of claims 1 through 5, wherein said NH₃ detection device (8) is a NOx sensor that detects NOx and NH₃ in the exhaust gas.

## Patentansprüche

1. System zur Bestimmung von Katalysatorverschleiß, das den Verschleiß eines NOx-Speicherreduktionskatalysators (4) bestimmt, der in einem Abgaskanal einer Brennkraftmaschine derart angeordnet ist, dass er NOx speichert und das darin gespeicherte NOx mittels der Zufuhr eines Reduktionsmittels reduziert, wobei das System umfasst:
eine Zuführeinrichtung (5), die das Reduktionsmittel dem NOx-Speicherreduktionskatalysator zuführt, um dadurch ein Luft-Kraftstoff-Verhältnis eines durch den NOx-Speicherreduktionskatalysator strömenden Abgases zu ändern;
eine Erfassungseinrichtung (8), die Mittel zum Erfassen von NH₃ im Abgas auf der stromabwärts liegenden Seite des NOx-Speicherreduktionskatalysators umfasst;
eine Steuereinrichtung (10), die Mittel zum Durchführen einer ersten Zufuhr des Reduktionsmittels und einer zweiten Zufuhr des Reduktionsmittels in aufeinanderfolgender Weise umfasst, indem eine Menge des Reduktionsmittels derart angepasst wird, dass zum Zeitpunkt der Zufuhr des Reduktionsmittels von der Zuführeinrichtung das Luft-Kraftstoff-Verhältnis des Abgases zu einem fetten Luft-Kraftstoff-Verhältnis wird; und
eine Bestimmungseinrichtung (10) umfassend Mittel zum Bestimmen, basierend auf dem erfassten Wert der Erfassungseinrichtung, ob der NOx-Speicherreduktionskatalysator einem Verschleiß erfahren hat, nachdem ein Zeitraum, der erforderlich ist damit die Menge an NH₃, die erzeugt wird durch die Reaktion des NOx im Abgas und des Reduktionsmittels nachdem die Reduktion des in dem NOx-Speicherreduktionskatalysator gespeicherten NOx vollendet wurde, zu einer Menge wird, die dem Ausmaß des Verschleiß des NOx-Speicherreduktionskatalysators entspricht, verstrichen ist seit dem Beginn der ersten Zufuhr des Reduktionsmittels und nach dem Beginn der zweiten Zufuhr des Reduktionsmittels.

2. System zur Bestimmung von Katalysatorverschleiß nach Anspruch 1, wobei die Steuereinrichtung (10) Mittel zum Anpassen der Menge an Reduktionsmittel derart, dass das Luft-Kraftstoff-Verhältnis des Abgases zum Zeitpunkt der zweiten Zufuhr des Reduktionsmittels magerer wird als zum Zeitpunkt der ersten Zufuhr des Reduktionsmittels und fetter als ein stöchiometrisches Luft-Kraftstoff-Verhältnis, umfasst.

3. System zur Bestimmung von Katalysatorverschleiß nach Anspruch 1, wobei die Steuereinrichtung (10) Mittel zum mehrmaligen Zuführen des Reduktionsmittels zum Zeitpunkt der zweiten Zufuhr des Reduktionsmittels, so dass eine Veränderung des Luft-Kraftstoff-Verhältnisses des Abgases zwischen einem fetten Zustand und einem mageren Zustand bewirkt wird, umfasst und einen Zeitraum während dem das Luft-Kraftstoff-Verhältnis des Abgases zu einem fetten Luft-Kraftstoff-Verhältnis wird für eine Zeit zum Zeitpunkt der zweiten Zufuhr des Reduktionsmittels kürzer macht als einen Zeitraum während dem das Luft-Kraftstoff-Verhältnis des Abgases zu einem fetten Luft-Kraftstoff-Verhältnis wird zum Zeitpunkt der ersten Zufuhr des Reduktionsmittels.

4. System zur Bestimmung von Katalysatorverschleiß nach einem der Ansprüche 1 bis 3, wobei, wenn der erfasste Wert der NH₃-Erfassungseinrichtung (8) gleich oder größer als ein Grenzwert ist, die Bestimmungseinrichtung (10) eine Bestimmung macht, dass der NOx-Speicherreduktionskatalysator (4) einen Verschleiß erfahren hat.

5. System zur Bestimmung von Katalysatorverschleiß nach einem der Ansprüche 1 bis 3, wobei, wenn der erfasste Wert der Erfassungseinrichtung (8) geringer als ein Grenzwert ist, die Bestimmungseinrichtung (10) eine Bestimmung macht, dass der NOx-Speicherreduktionskatalysator (4) einen Verschleiß erfahren hat.

6. System zur Bestimmung von Katalysatorverschleiß nach einem der Ansprüche 1 bis 5, wobei die NH₃-Erfassungseinrichtung (8) ein NOx-Sensor ist, der NOx und NH₃ im Abgas erfasst.

## Revendications

1. Système de détermination de la détérioration d'un catalyseur qui détermine la détérioration d'un catalyseur réducteur (4) de stockage de NOₓ qui est disposé dans une sortie d'échappement d'un moteur à combustion interne de telle manière qu'il stocke les NOₓ et réduit les NOₓ qui y sont stockés par la fourniture d'un réducteur, ledit système comprenant :
un dispositif d'alimentation (5) qui alimente en réducteur ledit catalyseur réducteur de stockage de NOₓ pour modifier de ce fait le rapport air/carburant d'un gaz d'échappement traversant ledit catalyseur réducteur de stockage de NOₓ ;
un dispositif de détection (8) comprenant un moyen de détecter le NH₃ dans le gaz d'échappement en aval dudit catalyseur réducteur de stockage de NOₓ ;
un dispositif de commande (10) comprenant un moyen d'effectuer un premier apport du réducteur et un second apport du réducteur de façon séquentielle en réglant la quantité du réducteur de telle manière que le rapport air/carburant du gaz d'échappement devient un rapport air/carburant riche au moment de la fourniture du réducteur provenant dudit dispositif d'alimentation ; et
un dispositif de détermination (10) comprenant un moyen de déterminer, sur la base de la valeur détectée par ledit dispositif de détection, si ledit catalyseur réducteur de stockage de NOₓ s'est détérioré, après que se soit écoulée, à partir du début dudit premier apport du réducteur et après le début dudit second apport du réducteur, la période qui est nécessaire pour que la quantité de NH₃ produite par la réaction entre les NOₓ dans le gaz d'échappement et le réducteur, après achèvement de la réduction des NOₓ stockés dans ledit catalyseur réducteur de stockage de NOₓ, devienne une quantité correspondant à l'étendue de la détérioration dudit catalyseur réducteur de stockage de NOₓ.

2. Système de détermination de la détérioration d'un catalyseur selon la revendication 1, dans lequel ledit dispositif de commande (10) comprend un moyen pour régler la quantité de réducteur de telle manière que le rapport air/carburant du gaz d'échappement au moment du second apport du réducteur devienne plus pauvre qu'au moment du premier apport du réducteur et plus riche qu'un rapport air/carburant stoechiométrique.

3. Système de détermination de la détérioration d'un catalyseur selon la revendication 1, dans lequel ledit dispositif de commande (10) comprend un moyen pour fournir le réducteur une pluralité de fois au moment du second apport du réducteur de sorte que l'on fait varier le rapport air/carburant du gaz d'échappement entre un niveau riche et un niveau pauvre et rend la période dans laquelle le rapport air/carburant du gaz d'échappement devient un rapport air/carburant riche pour une fois au moment dudit second apport du réducteur plus courte que la période dans laquelle le rapport air/carburant du gaz d'échappement devient un rapport air/carburant riche au moment dudit premier apport du réducteur.

4. Système de détermination de la détérioration d'un catalyseur selon l'une quelconque des revendications 1 à 3, dans lequel, quand la valeur détectée par ledit dispositif de détection (8) de NH₃ est supérieure ou égale à une valeur seuil, ledit dispositif de détermination (10) fait une constatation suivant laquelle ledit catalyseur réducteur (4) de stockage de NOₓ s'est détérioré.

5. Système de détermination de la détérioration d'un catalyseur selon l'une quelconque des revendications 1 à 3, dans lequel, quand la valeur détectée par ledit dispositif de détection (8) est inférieure à une valeur seuil, ledit dispositif de détermination (10) fait une constatation suivant laquelle ledit catalyseur réducteur de stockage de NOₓ s'est détérioré.

6. Système de détermination de la détérioration d'un catalyseur selon l'une quelconque des revendications 1 à 5, dans lequel ledit dispositif de détection (8) de NH₃ est une sonde de NOₓ qui détecte les NOₓ et le NH₃ dans le gaz d'échappement.
